(19) Europäisches Patentamt
European Patent Office
Office européen des brevets

(11) Veröffentlichungsnummer: **0 213 108**
**A2**

(12) **EUROPÄISCHE PATENTANMELDUNG**

(21) Anmeldenummer: 86890193.5

(22) Anmeldetag: 26.06.86

(51) Int. Cl.4: A61K 31/135 , A61K 9/72 , A61K 47/00

(30) Priorität: 26.06.85 AT 1909/85

(43) Veröffentlichungstag der Anmeldung: 04.03.87 Patentblatt 87/10

(84) Benannte Vertragsstaaten: BE CH DE FR GB IT LI LU NL SE

(71) Anmelder: Burghart, Kurt Dr.
Sägeberg 8
D-2217 Rosdorf(DE)
Anmelder: Burghart, Walter
Salmgasse 4
A-1030 Wien(AT)

(72) Erfinder: Burghart, Kurt Dr.
Sägeberg 8
D-2217 Rosdorf(DE)
Erfinder: Burghart, Walter
Salmgasse 4
A-1030 Wien(AT)

(74) Vertreter: Haffner, Thomas M., Dr.
Patentanwaltskanzlei Dipl.-Ing. Adolf
Kretschmer Dr. Thomas M. Haffner
Schottengasse 3a
A-1014 Wien(AT)

(54) **Pharmazeutische Zubereitung mit einem Antihypotonikum als wirksames Mittel.**

(57) Das α-und/oder $\beta_1$-Sympathomimetikum enthält als wirksames Mittel eine Verbindung der allgemeinen Formel

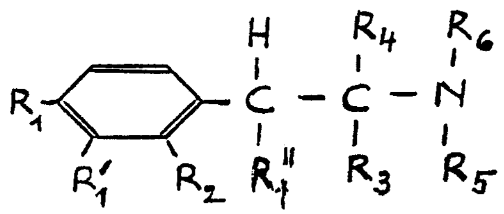
, (I)

in welcher $R_1$, $R'_1$, $R''_1$ und $R_2$ identisch oder voneinander verschieden H , OH oder Methoxy bedeuten und wenigstens einer dieser Reste OH und wenigstens einer der Ringsubstituenten OH oder $OCH_3$ bedeutet, $R_3$, $R_4$ und $R_6$ jeweils gleich oder voneinander verschieden Wasserstoff, eine Methyl- oder eine Äthylgruppe bedeuten, und $R_5$ Wasserstoff, eine Methylgruppe, oder eine Äthylgruppe, und im Falle der Bedeutung von H für $R_5$ und $CH_3$ für $R_4$ nur einer der Ringsubstituenten OH bedeutet, als freie Base oder wenigstens teilweise als Salz einer organischen Säure, wie Fett-, Sorbin-,

EP 0 213 108 A2

Stearin-, Wein-, Zitronen-oder Ascorbinsäure, gelöst vorliegt, und ist unter Pumpen-oder Treibgasdruck sublingual oder inhalatorisch applizierbar.

## Pharmazeutische Zubereitung mit einem Antihypotonikum als wirksames Mittel

Die Erfindung bezieht sich auf eine pharmazeutische Zubereitung mit einem Antihypotonikum, insbesondere einem α -und/oder $\beta_1$-Sympathomimetikum, als wirksames Mittel.

Sympathomimetika sind Stoffe, die Symptome auslösen, die denjenigen mehr oder weniger entsprechen, die durch eine Stimulation der sympathischen Nerven hervorgerufen werden. Als Sympathomimetika sind vor allen Dingen die körpereigenen Sympathomimetika Adrenalin und Noradrenalin bekannt. Auf der Basis dieser beiden bekannten Amine sind eine Reihe von synthetischen Sympathomimetika entwickelt worden, welche eine enge Verwandtschaft mit dem genannten Verbindungen aufweisen und als Derivate des β-Phenyläthylamins bezeichnet werden können. Neben Verbindungen mit phenolischen OH-Gruppen sind auch Phenylalkylamine ohne phenolische Hydroxylgruppen, im besonderen Ephedrin und Ephedrinderivate, bekannt.

Der Einsatz derartiger Präparate zielt zumeist auf eine Behandlung von Kreislaufstörungen auf Grund der vasokonstriktorischen sowie der die Herzfrequenz stimulierenden Wirkung der Wirkstoffe sowie auf eine Behandlung von Allergien, wie Heuschnupfen, oder auf die Behandlung von Asthma, ab. Insbesondere im erstgenannten Fall der Behandlung von Kreislaufschwächen kommt der raschen Bioverfügbarkeit besondere Bedeutung zu. Die bekannten Verbindungen werden zumeist in Form ihrer Hydrochloride oral verabreicht, wobei zumeist ein relativ langer Zeitraum bis zur Wirksamkeit beobachtet wird. Relativ rasche Wirksamkeit läßt sich durch intramuskuläre oder intravenöse Verabreichung, beispielsweise von Dopamin-HCl bei Kreislaufschock erzielen, jedoch ist man bei derartigen Darreichungen auf ärztliche Hilfe angewiesen. Auch ist beispielsweise bei oraler Verabreichung von 1-(3'–Hydroxyphenyl)-2-aminoäthanol-hydrochlorid die sichere orale Wirkung teilweise umstritten und in der Regel frühestens nach 10 bis 15 min zu erwarten. In der Regel werden die bekannten Sympathomimetika nicht zuletzt deshalb als Hydrochloride eingesetzt, weil die Hydrochloride wesentlich stabiler sind und geringere Anforderungen an den Lichtschutz und den Schutz gegen Oxydation stellen.

Die Erfindung zielt nun darauf ab, eine pharmazeutische Zubereitung der eingangs genannten Art zu schaffen, welche auch ohne intramuskuläre oder intravenöse Verabreichung nach relativ kurzer Zeit bereits eine deutliche Wirkung zeigt. Zur Lösung dieser Aufgabe besteht die Erfindung im wesentlichen darin, daß eine Verbindung der allgemeinen Formel

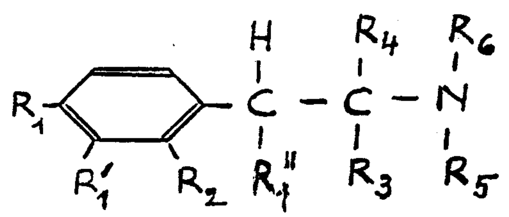

, (I)

in welcher $R_1$, $R'_1$, $R''_1$ und $R_2$ identisch oder voneinander verschieden H , OH oder Methoxy bedeuten und wenigstens einer dieser Reste OH und wenigstens einer der Ringsubstituenten OH oder $OCH_3$ bedeutet, $R_3$, $R_4$ und $R_6$ jeweils gleich oder voneinander verschieden Wasserstoff, eine Methyl- oder eine Äthylgruppe bedeuten, und $R_5$ Wasserstoff, eine Methylgruppe oder eine Äthylgruppe, und im Falle der Bedeutung von H für $R_5$ und $CH_3$ für $R_6$ nur einer der Ringsubstituenten OH bedeutet, als freie Base oder wenigstens teilweise als Salz einer organischen Säure, wie Fett-, Sorbin-, Stearin-, Wein-, Zitronen-oder Ascorbinsäure, gelöst vorliegt und unter Pumpen-oder Treibgasdruck sublingual oder inhalatorisch applizierbar ist.

Dadurch, daß die freie Base oder ein Salz einer organischen Säure Verwendung findet, läßt sich die Resorption bei einer Darreichung als Aerosol wesentlich beschleunigen und es kann gegenüber den bekannten oralen Darreichungen der entsprechenden Hydrochloride gemäß dem bekannten Stand der Technik ein sicherer Wirkungseintritt bereits innerhalb von 3 min beobachtet werden.

Überraschenderweise hat sich hiebei gezeigt, daß geringe Mengen organischer Salze die Stabilität und das Lösungsverhältnis wesentlich verbessern, wobei ein gegenüber Hydrochloriden wesentlich rascherer Wirkungseintritt beobachtet wird. Die Probleme mit der Stabilität, insbesondere gegen Oxydation, und dem erforderlichen Lichtschutz, lassen sich vor allen Dingen bei Sprays, auf Grund der lichtundurchlässigen, treibgasgefüllten Dosen, insbesondere aber auch durch Zusatz von Stabilisierungsmitteln, wie α-Tocopherolen, Butyl-hydroxyanisol oder -toluol und anderen Antioxidantien leicht beherrschen.

In besonders vorteilhafter Weise werden als wirksames Mittel β-Phenyläthylaminderivate, Noradrenalin und/oder Norfenefrin und/oder Etilefrin in alkoholischer Lösung, insbesondere in Äthanol und/oder Polyäthylenglykol und/oder Propylenglykol und/oder in einer Lösung von Triglyceriden mit kurzer oder mittlerer Kettenlänge, wie Triazetin oder Miglyol und gegebenenfalls Wasser bzw. deren Gemischen vorliegt, eingesetzt. Die freie Base ist zum Unterschied vom entsprechenden Hydrochlorid, wie es üblicherweise bei oraler Verabreichung eingesetzt wird, in Wasser schlechter löslich. In äthanolischer Lösung konnte jedoch eine wesentlich raschere Wirksamkeit und ein rascherer physiologischer Wirkungseintritt nach dem Einsprühen in den Mundraum eines Patienten beobachtet werden. Die Lösbarkeit läßt sich durch Verwendung von 1,2-Propylenglykol als Lösungsvermittler wesentlich verbessern, was im besonderen bei Verwendung von 1-(3'-Hydroxyphenyl)-2-aminoäthanol als wirksames Mittel von Bedeutung ist. Mit besonderem Vorteil läßt sich aber auch als wirksames Mittel 1-(3'-Hydroxyphenyl)-2-äthylaminoäthanol, insbesondere in Verbindung mit einer Fettsäure, einsetzen.

Für die erfindungsgemäße Darreichung der pharmazeutischen Zubereitung wird das wirksame Mittel als Aerosol, insbesondere unter Verwendung von halogenierten Kohlenwasserstoffen wie Chlorfluorkohlenwasserstoffen als Treibgas, vorrätig gehalten.

Für eine sichere Dosierung bei entsprechenden Aerosolmengen enthalten die Lösungen mit Vorteil 1 bis 20 Gew.-%, bezogen auf die gesamte Lösung, an freier Base.

Um eine stabile Lösung zu erzielen, wird die Zubereitung mit Vorteil so vorgenommen, daß bei Verwendung von Fettsäuren zur wenigstens teilweisen Salzbildung, insbesondere von Stearin-oder Sorbinsäure, ein Gewichtsverhältnis 2:1 mg freie Base : mg Fettsäure eingestellt wird, wobei vorzugsweise die zugegebene Menge Fettsäure geringer ist als die stöchiometrische Menge für eine vollständige Salzbildung. Bei Verwendung von anderen organischen Säuren wie Weinsäure, Zitronensäure oder Ascorbinsäure wird mit Vorteil das Gewichtsverhältnis mg Base zu mg Säure kleiner als 3:1 gewählt. Zur Untersuchung der Stabilität der Lösungen wurden nachfolgende Gemische zubereitet:

| | Versuch 1 | Versuch 2 | Versuch 3 | Versuch 4 | Versuch 5 |
|---|---|---|---|---|---|
| Etilefrin-base | 2,1mg | 2,1mg | 2,1mg | 2,1mg | 2,1mg |
| Stearin-säure | 2,5mg | 2,0mg | 1,5mg | 1,0mg | 0,5mg |
| Äthanol | 56,0mg | 56,0mg | 56,0mg | 56,0mg | 56,0mg |
| Miglyol | 30,0mg | 30,0mg | 30,0mg | 30,0mg | 30,0mg |
| TRG12 | 59,4mg | 59,9mg | 60,4mg | 60,9mg | 61,4mg |

Die Versuche 1 bis 4 ergeben vollständige Löslichkeit, wohingegen bei Versuch 5 festgestellt wurde, daß die Lösbarkeit nicht mehr gegeben ist. Als Treibgas wurde Dichlorfluormethan verwendet. Der pH-Wert der in den Versuchen 1 bis 5 hergestellten Lösungen lag zwischen 6 und 5,5. Bei Versuch 5 wurde ein Ausfallen von Etilefrin bei 4°C beobachtet.

Die raschere Wirksamkeit der erfindungsgemäßen pharmazeutischen Zubereitung relativ zu einer konventionellen Zubereitung wird nachfolgen an Hand eine Ausführungsbeispieles näher erläutert.

In einer alkoholischen Lösung wurden hiebei unter Verwendung von Trichlorfluorkohlenwasserstoff als Treibgas 2,5 mg 1-(3'--Hydroxyphenyl)-2-aminoäthanol als Aerosol sublingual verabreicht. In der nachfolgenden Tabelle I sind unter A die Vergleichswerte für den systolischen und den diastolischen Druck über die Zeit bei Versprühen eines

Placebos, unter B die Werte für den systolischen und den diastolischen Druck über die Zeit bei Verabreichung von 2,5 mg Base als Aerosol und unter C die systolischen und diastolischen Druckwerte bei Verabreichung der gleichen Wirksubstanz als Hydrochlorid und oral über die Zeit aufgetragen, wobei mit dem Zeitpunkt 0 Referenzwerte vorangestellt sind. Aus Tabelle B ist hiebei der rasche diastolische Druckanstieg bei sublingualer Verabreichung als Aerosol ersichtlich, wohingegen aus Tabelle C eine Steigerung des diastolischen Drucks frühestens nach Ablauf von 15 min entnommen werden kann.

Tabelle I

| | Zeit min | Puls $min^{-1}$ | Systole kPa | Diastole kPa |
|---|---|---|---|---|
| A | | | | |
| | 1' | 66 | 14,80 | 8,53 |
| | 3' | 64 | 15,20 | 7,99 |
| | 5' | 61 | 14,53 | 8,80 |
| | 7' | 65 | 14,67 | 7,99 |
| | 10' | 64 | 14,40 | 8,93 |
| | 12' | 61 | 14,13 | 8,80 |
| | 15' | 61 | 15,73 | 7,99 |
| B | | | | |
| | 1' | 71 | 15,20 | 9,33 |
| | 3' | 63 | 14,67 | 9,20 |
| | 5' | 69 | 14,53 | 8,27 |
| | 7' | 66 | 14,80 | 9,20 |
| | 10' | 64 | 14,93 | 8,93 |
| | 12' | 63 | 15,33 | 9,33 |
| | 15' | 61 | 14,53 | 8,67 |
| | 0' | 68 | 14,67 | 8,93 |
| | 0' | 67 | 14,27 | 8,53 |
| | 0' | 65 | 14,40 | 8,67 |
| C | 1' | 64 | 14,40 | 8,53 |
| | 3' | 66 | 14,00 | 8,67 |
| | 5' | 71 | 14,27 | 8,53 |
| | 7' | 68 | 14,40 | 8,53 |
| | 10' | 66 | 14,67 | 8,80 |
| | 12' | 68 | 13,87 | 8,80 |
| | 15' | 77 | 14,40 | 8,67 |
| | 20' | 52 | 14,93 | 9,33 |
| | 25' | 67 | 13,73 | 8,80 |
| | 30' | 69 | 14,27 | 9,60 |

**Ansprüche**

1. Pharmazeutische Zubereitung mit einem Antihypotonikum, insbesondere einem α-und/oder β,-Sympathomimetikum, als wirksames Mittel, dadurch gekennzeichnet, daß eine Verbindung der allgemeinen Formel

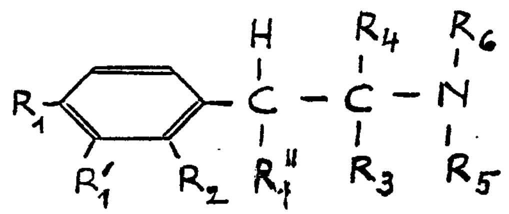

, (I)

in welcher $R_1$, $R'_1$, $R''_1$ und $R_2$ identisch oder voneinander verschieden H , OH oder Methoxy bedeuten und wenigstens einer dieser Reste OH und wenigstens einer der Ringsubstituenten OH oder $OCH_3$ bedeutet, $R_3$, $R_4$ und $R_6$ jeweils gleich oder voneinander verschieden Wasserstoff, eine Methyl- oder eine Äthylgruppe bedeuten, und $R_5$ Wasserstoff, eine Methylgruppe, oder eine Äthylgruppe, und im Falle der Bedeutung von H für $R_5$ und $CH_3$ für $R_4$ nur einer der Ringsubstituenten OH bedeutet, als freie Base oder wenigstens teilweise als Salz einer organischen Säure, wie Fett-, Sorbin-, Stearin-, Wein-, Zitronen-oder Ascorbinsäure, gelöst vorliegt und unter Pumpen-oder Treibgasdruck sublingual oder inhalatorisch applizierbar ist.

2. Pharmazeutische Zubereitung nach Anspruch 1, dadurch gekennzeichnet, daß als wirksames Mittel β-Phenyläthylaminderivate, Adrenalin und/oder Noradrenalin und/oder Norfenefrin und/oder Etilefrin in alkoholischer Lösung, insbesondere in Äthanol und/oder Polyäthylenglykol und/oder Propylenglykol und/oder in einer Lösung von Triglyceriden mit kurzer oder mittlerer Kettenlänge, wie Triazetin oder Miglyol und gegebenenfalls Wasser bzw. deren Gemischen vorliegt.

3. Pharmazeutische Zubereitung nach Anspruch 1 oder 2, dadurch gekennzeichnet, daß das wirksame Mittel zur Darreichung als Aerosol, insbesondere unter Verwendung von halogenierten Kohlenwasserstoffen wie Chlorfluorkohlenwasserstoffen als Treibgas, vorliegt.

4. Pharmazeutische Zubereitung nach Anspruch 2, dadurch gekennzeichnet, daß die Lösung 1 bis 20 Gew.-% bezogen auf die gesamte Lösung der freien Base bzw. des Salzes einer organischen Säure enthält.

5. Pharmazeutische Zubereitung nach einem der Ansprüche 1 bis 4, dadurch gekennzeichnet, daß als wirksames Mittel 1-(3'-Hydroxyphenyl)-2-aminoäthanol eingesetzt ist.

6. Pharmazeutische Zubereitung nach einem der Ansprüche 1 bis 4, dadurch gekennzeichnet, daß als wirksames Mittel 1-(3'-Hydroxyphenyl)-2-äthylaminoäthanol eingesetzt ist.

7. Pharmazeutische Zubereitung nach einem der Ansprüche 1 bis 6, dadurch gekennzeichnet, daß bei Verwendung von Fettsäuren zur wenigstens teilweisen Salzbildung, insbesondere von Stearin-oder Sorbinsäure, ein Gewichtsverhältnis von wenigstens 2:1 mg freie Base : mg Fettsäure eingestellt wird.

8. Pharmazeutische Zubereitung nach Anspruch 7, dadurch gekennzeichnet, daß die zugegebene Menge Fettsäure geringer ist als die stöchiometrische Menge für eine vollständige Salzbildung.

9. Pharmazeutische Zubereitung nach einem der Ansprüche 1 bis 6, dadurch gekennzeichnet, daß bei Verwendung von anderen organischen Säuren, wie Weinsäure, Zitronensäure oder Ascorbinsäure, das Gewichtsverhältnis mg Base zu mg Säure kleiner als 3:1 gewählt wird.

10. Pharmazeutische Zubereitung nach einem der Ansprüche 1 bis 9, dadurch gekennzeichnet, daß 1,2-Propylenglykol als Lösungsvermittler zugesetzt ist.